# EUROPEAN PATENT SPECIFICATION

(11) **EP 1 205 182 B1**
(45) Date of publication and mention of the grant of the patent: **13.07.2005**
(21) Application number: 01112092.0
(22) Date of filing: 29.05.2001
(51) Int. Cl.: A61K 31/7076, A61K 31/00, A61K 38/58, A61K 31/727, A61P 7/02, A61P 9/10

(54) **Adenosine as antithrombotic**
Adenosine als Antithrombosemittel
Adénosine en tant qu'antithrombotique

(30) Priority: 07.11.2000 US 708306
(43) Date of publication of application: 15.05.2002
(73) Proprietor: Advanced Gene Technology, Corp., 407 Taichung (TW)
(72) Inventor: Chang, Su-Chen, 406 Taichung (TW); Hsu, Li-Wei, 406 Taichung (TW)
(74) Representative: Albrecht, Thomas, Dr.

(56) References cited:
- WO-A-99/02143
- US-A- 5 677 290
- US-A- 5 976 532
- US-A- 6 137 002
- KUTSUNA H ET AL: "Identification and Determination of Platelet Aggregation Inhibitor from Safflower (Carthamus tinctorius Linne)" YAKUGAZU ZASSHI, vol. 108, no. 11, 1988, pages 1101-1103, XP001080271
- OKUYAMA T ET AL: "Effects of Chinese Drugs "Yiebai" and "Dasuan" on human platelet aggregation (Allium bajeri, A. Sativum)" PLANTA MEDICA, vol. 55, no. 3, 1989, pages 242-244, XP001085484
- ZENNI C ET AL.: "Studies on the water soluble constituents of Chinese drug Belmu, the bulbs of Fritillaria plants." ZHONGGUO-ZHONGYAO-ZAZHI, vol. 21, no. 7, 1996, pages 420-422, 447, XP001085463
- CRISTALLI G ET AL.: "Inhibition of platelet aggregation by adenosine receptor agonists." NAUYN-SCHMIEDEBERGS ARCH PHARMACOL, vol. 349, 1994, pages 644-650, XP001083858
- DAWICKI DD: "Potentiation of the antiplatelet action of adenosine in whole blood by dipyridamole or dilazep and the cAMP phosphodiesterase inhibitor, RA233." THROMBOSIS RESEARCH, vol. 43, 1986, pages 161-175, XP001083857
- WARREN MK ET AL.: "Early suppressive effects of chemotherapy on recovery of bone marrow megakaryocyte precursors: possible relationship to platelet recovery." STEM CELLS, vol. 14, no. 1, 1996, pages 31-37, XP001083885
- PATENT ABSTRACTS OF JAPAN vol. 017, no. 129 (P-1503), 18 March 1993 (1993-03-18) & JP 04 309863 A (DAI ICHI PURE CHEM CO LTD), 2 November 1992 (1992-11-02)

## Description

### Technical Field of the Invention

The present invention relates to the novel use of adenosine as an antagonist against a platelet membrane receptor protein gpIIb/IIIa for inhibiting platelet aggregation and thrombosis.

### Background of the Invention

Most of the thromboembolic disorders, including atherosclerosis and arteriosclerosis, acute myocardial infarction, angina, transient ischemic attacks and strokes, peripheral vascular diseases, arterial thrombosis, preeclampsia, embolism and carotid endarterectomy, are related to the formation of blood clot or thrombus in blood vessel. Platelet aggregation plays an important role in the thrombus formation. It was found that platelet aggregation is dependent upon the binding of fibrinogen and other serum proteins to a platelet membrane receptor protein gpIIb/IIIa located on the platelet plasma membrane. When platelet is activated by an agonist such as thrombin, the gpIIb/IIIa binding site becomes available to fibrinogen and other serum proteins for binding, thereby resulting in the platelet aggregation and thrombus formation. Thus, inhibition of the binding of fibrinogen and other serum proteins to gpIIb/IIIa is a requisite for the prevention of thrombus formation.

The development of a gpIIb/IIIa antagonist represents a promising approach to inhibiting platelet aggregation and thrombosis. A gpIIb/IIIa-specific antiplatelet agent which can inhibit the activation and aggregation of platelets in response to any agonist is therefore required.

Various products such as aspirin against arachidonic acid, ticlopidine against adenosine diphosphate (ADP), hirudin against thrombin, and thromboxane A₂ synthase inhibitors or receptor antagonists against thromboxane A₂ synthase, are available now for preventing platelet aggregation and thrombus formation. However, these products do not specifically inhibit the binding of fibrinogen and other serum proteins to gpIIb/IIIa and also have a serious side effect of causing prolong bleeding.

USP 6,137,002, USP 6,020,362, USP 5,731,324 and USP 5,618,843 disclosed certain bicyclic compounds having a nucleus formed of two fused six membered rings, which were useful as gpIIb/IIIa antagonists for the inhibition of platelet aggregation.

USP 6,017,877, USP 6,013,625, USP 5,858,972, USP 5,780,303, USP 5,672,585 and USP 5,612,311 disclosed certain cyclic peptides having a high affinity for gpIIb/IIIa, which were useful in the inhibition of platelet aggregation and thus in the treatment of thrombosis.

USP 5,849,693, USP 5,773,411, USP 5,817,749, USP 5,668,159 and USP 5,635,477 disclosed certain cyclic compounds linked by a heterocyclic ring system, which were useful as gpIIb/IIIa antagonists for the treatment of thrombosis.

USP 5,053,393 disclosed N-[8-[(aminoiminomethyl)amino]-1-oxooctyl]-N-L-α-aspartyl-L-phenylalanine as a potent compound for the inhibition of platelet aggregation.

USP 4,879,313 disclosed certain peptide mimetic compounds, which were useful in the inhibition of platelet aggregation and in the treatment of thrombosis.

USP 5,951,981 disclosed certain chemically crosslinked conjugates of fibrolase and a specific binding peptide, which exhibited a thrombolytic activity.

USP 5,780,590 disclosed a compound N-[N-[N-[(4-piperdin-4-yl)butanoyl]-N-ethylglycyl]-(L)-aspartyl]-(L)-β-cyclohexyl-alanine amide as an antithrombotic.

USP 5,681,823 disclosed a compound P¹, P⁴-dithio-P², P³-monochloromethylene 5', 5'''-diadenosine P¹, P⁴-tetraphosphate as an antithrombotic.

Kutsuna et al (Yakugaku Zasshi, 108 (11), pp. 1101-1103 (1998)) describe the identification and determination of adenosine as platelet aggregation inhibitor from safflower (Carthamus tinctonus L.).

Okuyama et al. (Planta Medica, 55 (3), pp. 242-244 (1989)) describe the effects of chinese drugs "Xiebai" and "Dasuan" on human platelet aggregation and disclose that, inter alia, adenosine was isolated from the n-butanol-soluble fraction of Xiebai, which was recognized to have an anti-platelet aggregation effect.

Zenai et al. (Zhongguo-Zongyao-zahzi 21 (7), pp. 420-422, 447 (1996)) describe studies on the water soluble constituents of Chinese drug Beimu, the bulbs of *Fritillaria* plants and disclose that platelet aggregation assays showed that adenosine is the chief aggregation inhibitor.

Cristalli et al (Naunyn-Schmiedberg's Arch Pharmacol. 349, pp. 644-650 (1994)) describe the inhibition of platelet aggregation by adenosine receptor agonists and state that adenosine uncompetitively inhibits platelet aggregation initiated by agents such as ADP, vasopressin or collagen.

Dawicki et al. (Thrombosis Research 34, pp. 161-175 (1986)) describe the potentiation of the antiplatelet action of adenosine in whole blood by dipyridamole or dilazep and the cAMP phosphodiesterase inhibitor RA 233 and state that adenosine is a potent inhibitor of ADP-induced human platelet aggregation in platelet-rich plasma, but does not inhibit aggregation in whole blood.

International patent application WO 99/02143 discloses the use of adenosine and active agent which interacts with the adenosine system to treat conditions of weakened immune system, as anti-cancer therapy and for improving the therapeutic index of a variety of therapeutic drugs.

USP 5,677,290 describes a method of inducing anesthesia, sedation, analgesia, hypothermia, and reduced stress by administering an effective amount of an adenosine compound to a mammal and a method for preserving donor organs in vivo by contacting them with an adenosine compound, as well as a method for preparing organ recipients for transplants.

USP 6,137,002 describes certain bicyclic compounds having a nucleus formed of two fused six membered rings, for example, isoquinoline, isoquinolone, tetrahydronaphthalene, dihydronaphthalene or tetralone, substituted with both basic and acidic functionality, which are useful in inhibition of platelet aggregation.

USP 5,976,532 describes platelet-specific, chimeric immunoglobulin and immunoglobulin fragments which block ligand binding to the glycoprotein IIb/IIIa receptor and prevent platelet aggregation.

Warren et al. (Stem Cells 14 (Suppl. 1), pp 31-37 (1996)) describe the early suppressive effect of chemotherapy on recovery of bone marrow megakaryocyte precursors and the possible relationship to platelet recovery.

JP 04-309863 describes an anticoagulant/anti platelet agent composition comprising, inter alia, adenosine for extravascular blood and the measurement of a particle marker for activating platelets using said composition.

The inventors of the present invention applied the high-density gridding technology for the screening of fractions of an extract of *Carthamus tinctorius L* for active ingredients that can specifically bind to gpIIb/IIIA, and surprisingly found that a small compound, identified as adenosine, can strongly bind to gpIIb/IIIa and thus can inhibit platelet aggregation and thrombosis, especially in view of the fact that ADP was well known in the art as an endogenous agonist for activating platelets. Accordingly, the inventors of the present invention found that adenosine is a potent antithrombotic.

Adenosine, a compound well known in the art, was used as an antiarrhythmic, and its derivatives were used as anti-tumor agents. The therapeutic use of adenosine for treating thromboembolic disorders was never disclosed or suggested in any prior art reference.

Based on the above, the inventors of the present invention found a new therapeutic use or indication of adenosine in the inhibition of thrombus formation.

### Summary of the Invention

The present invention provides the use of adenosine for the preparation of a medicament for inhibiting thrombosis in mammals. The present invention also provides the use of adenosine for the preparation of a medicament for treating mammals for thromboembolic disorders including atherosclerosis and arteriosclerosis, acute myocardial infarction, angina, transient ischemic attacks and strokes, peripheral vascular diseases, arterial thrombosis, preeclampsia, embolism and carotid endarterectomy.

The high-density gridding technology was applied to the screening of fractions of an extract of *Carthamus tinctorius L* for active ingredients that can specifically bind to a platelet membrane receptor protein gpIIb/IIIA.

The present invention also relates to the novel use of adenosine as an antagonist against gpIIb/IIIa for inhibiting platelet aggregation and thrombosis.

### Brief Description of the Figures

Figure 1A shows the HPLC elution profile of an extract of *Carthamus tinctorius L* in 120 minutes.

Figure 1B shows the binding profile of the eluted samples shown in Figure 1A to the protein gpIIb/IIIa that was detected by the absorption at a wavelength of 405 nm.

Figure 2A shows the HPLC elution profile of a sample pool collected from the fractions No. 25 to 40 shown in Figure 1A in 20 minutes.

Figure 2B shows the binding profile of the eluted samples shown in Figure 2A to the protein gpIIb/IIIa that was detected by the absorption at a wavelength of 405 nm.

Figure 3 shows the HPLC elution profile of a sample pool collected from the fractions No 5 and 6 shown in Figure 2A in 15 minutes, indicating that there is a single peak with a retention time of 10.7 minutes which shows the strongest binding activity to the protein gpIIb/IIIa.

Figure 4A shows the molecular weight 268 gm/mole of the compound obtained from the profile of Figure 3 that was detected by the electrospray ionization mass spectrometry.

Figure 4B shows the presence of sodium salt and oligomers (2-mer to 7-mer) of the compound obtained from the profile of Figure 3.

Figure 5 shows the binding curve of the compound obtained from Example 4 with the protein gpIIb/IIIa, indicating that the binding dramatically increased up to 80 % of the maximum binding when the concentration of the compound is below 10 µg/ml.

Figure 6A shows a dose response of the compound obtained from Example 4 in the inhibition of platelet aggregation, indicating that the maximum inhibition activity is about 85 % when the concentration of the compound is from 10 to 15 µg/ml blood.

Figure 6B shows the inhibition activity on platelet aggregation in a time course manner when the concentration of the compound obtained from Example 4 is 17.4 µg/ml, indicating that about 85 % of the maximum inhibition activity occurs at a period from 14^{th} to 16^{th} minutes.

Figure 7A shows the inhibition of thrombus formation *in vivo* in mesenteric vein of rats by administering the compound (400 µg/100 µl/rat) obtained from Example 4 in a time course manner.

Figure 7B shows a dose response of the compound obtained from Example 4 in the inhibition of thrombus formation *in vivo* in mesenteric vein of rats by intravenous injection.

Figure 7C shows a dose response of the compound obtained from Example 4 in the inhibition of thrombus formation *in vivo* in mesenteric vein of rats by oral administration.

Figure 8 shows a result of a competitive ELISA assay using each of the compound obtained from Example 4, commercial adenosine and ReoPro® (abciximab) for inhibiting the binding of fibrinogen to gpIIb/IIIa.

### Detailed Description of the Invention

The present invention relates to the novel use of adenosine as an antagonist against a platelet membrane receptor protein gpIIb/IIIa for inhibiting thrombosis.

It was a discovery of the present invention that an active ingredient obtained from the fractions of an extract of *Carthamus tinctorius L* by employing the high-density gridding technology could specifically bind to gpIIb/IIA, which was then identified as adenosine. Thus, adenosine is useful for the inhibition of platelet aggregation and thrombosis.

The high-density gridding technology has been used in the art for qualitatively or quantitatively detecting the presence of a target material in biological samples. The high-density gridding technology immobilizes arrays of biological samples in a small or even tiny volume on a gridding surface of a solid support. In particular, the biological samples suspected of containing target material are fixed or immobilized on a solid support, a labeled probe that can hybridize with or conjugate to the target material is added onto the solid support, the hybridized or conjugated solid support is then processed, imaged and analyzed, and the candidate target material that specifically interacts with the labeled probe can be quickly selected through screening.

An extract of *Carthamus tinctorius L* was fractionated by HPLC. Individual fractions were allocated on a plastic plate. A labeled platelet membrane receptor protein gpIIb/IIIa, an important factor involved in the platelet aggregation and thrombus formation, was added to the plastic plate for binding. The unbound gpIIb/IIIa was then stripped off. The candidate fractions showing a signal of binding to the labeled gpIIb/IIIa were selected, and the previous steps were repeated until a single ingredient interacting with the labeled gpIIb/IIIa was obtained. The single ingredient that can specifically bind to gpIIb/IIIa was identified as adenosine.

Adenosine was a compound well known in the art for use as an antiarrhythmic, and its derivatives were used as anti-tumor agents. The therapeutic use of adenosine for specifically binding to gpIIb/IIIa and treating thromboembolic disorders was never disclosed or suggested in any prior art reference. Thus, the present invention discloses that adenosine is useful as an antithrombotic for treating thromboembolic disorders.

The thromboembolic disorders includes, for examples, atherosclerosis and arteriosclerosis, acute myocardial infarction, angina, transient ischemic attacks and strokes, peripheral vascular diseases, arterial thrombosis, preeclampsia, embolism and carotid endarterectomy.

The present invention provides a method for inhibiting thrombosis by administering to a mammal including human an effective amount of adenosine. The present invention also provides a method for treating thromboembolic disorders by administering to a mammal including human an effective amount of adenosine.

Pharmaceutical compositions can be administered orally or by intravenous injection (i.v.). The suitable dosage form of the pharmaceutical composition includes, for example, tablets, capsules, pills, powders, granules, solution, elixirs, tinctures, suspensions, syrups, emulsions, and the likes.

The dosage of the pharmaceutical compositions will vary in consideration of the route of administration, the age, health, physical conditions and body weight of the recipients, the nature and extent of symptoms, and the effect desired. By way of general guidance, the daily oral dosage of adenosine will range between about 15 to 150 mg/kg of body weight, and the daily i.v. dosage of adenosine will range between about 1.5 to 15 mg/kg of body weight.

The pharmaceutical compositions can be administered in combination with other well known antithrombotics for achieving a synergetic effect in therapy. The well known antithrombotics are, for example, coumarin, aspirin, heparin, LMW heparin, ticlopidine, hirudin, and thromboxane A₂ synthase inhibitors or receptor antagonists.

The pharmaceutical composition tested showed no acute toxicity to the recipients in view of the data obtained from animal (rat) model where a single dosage amount of adenosine of approximately up to 70 mg/kg was administered intravenously.

The following Examples are provided to further illustrate the present invention.

### Examples

### Example 1

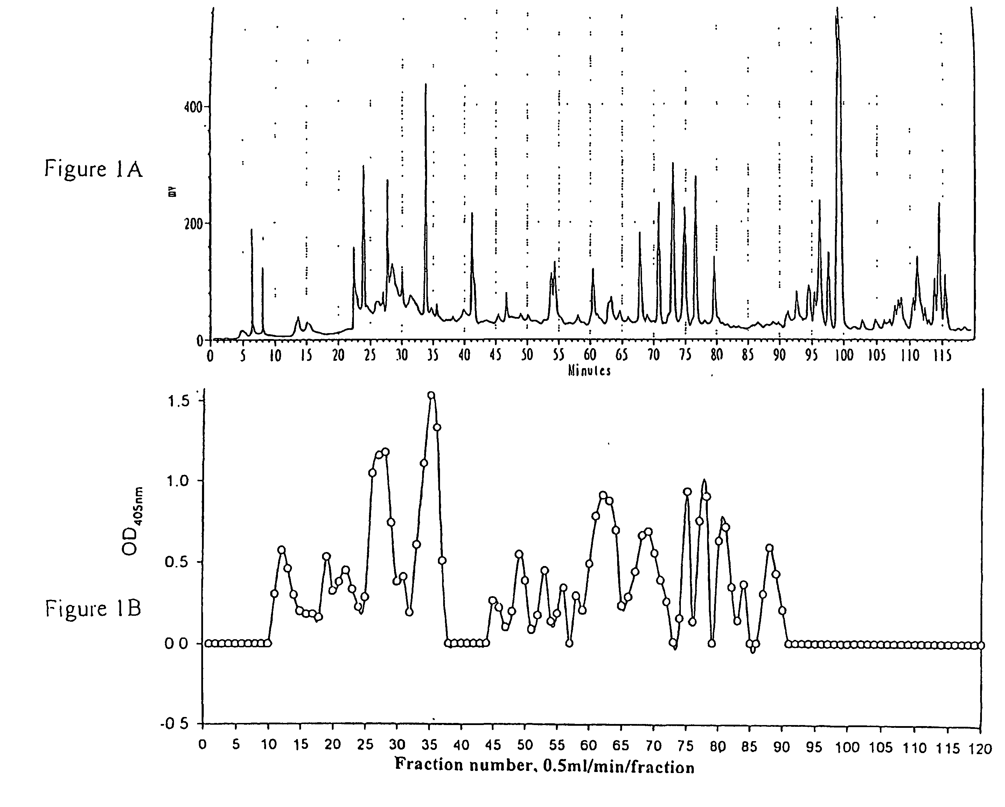

Five gram of *Carthamus tinctorius L* (purchased from Uni Chinese Herb Store, Taichung, Taiwan) was extracted with methanol (40 ml) by regular blending. The extract was concentrated to a final volume of 8 ml. For conducting HPLC (Shimadzu 10-AT, Japan) analysis, the concentrated extract (100 µl) was injected into ODS-gel ODS 80TM (4.6 mm×25 cm, TOSOH, Japan) column. The concentrated extract was first eluted with water for 5 minutes, followed by elution with an ethanol-water eluant with a linear increase of ethanol concentration from 0 to 70 % (v/v) within 105 minutes. In the next 5 minutes for elution, the concentration of ethanol in the eluant was increased to 100 % (v/v). The eluted samples were detected at a wavelength of 254 nm and were collected every 0.5 ml. The elution profile was shown in Figure 1A.

For conducting the target-binding assay, platelet membrane receptor protein gpIIb/IIIa was purified from platelets, and its purity was determined by SDS-PAGE and silver staining. The purified protein gpIIb/IIIa was labeled with biotin as described in the conventional protocol. The 120 collected samples were individually coated onto a 384-well plastic plate. The biotin-labeled protein gpIIb/IIIa was added to the sample-coated plastic plate and was incubated with the coated samples at room temperature for 30 minutes. Each well was then washed with TBST buffer for 3 times, to which the extravidin-conjugated alkaline phosphatase was added, and each well was further incubated for 30 minutes. The previous washing step was repeated and each well was then colored with substrate p-nitrophenyl phosphate. The absorption for each well at a wavelength of 405 nm was determined by an automatic ELISA reader (Dynax). Figure 1B showed the OD₄₀₅ₙₘ value of the samples corresponding to those shown in Figure 1A, indicating their capacity of binding to the protein gpIIb/IIIa.

### Example 2

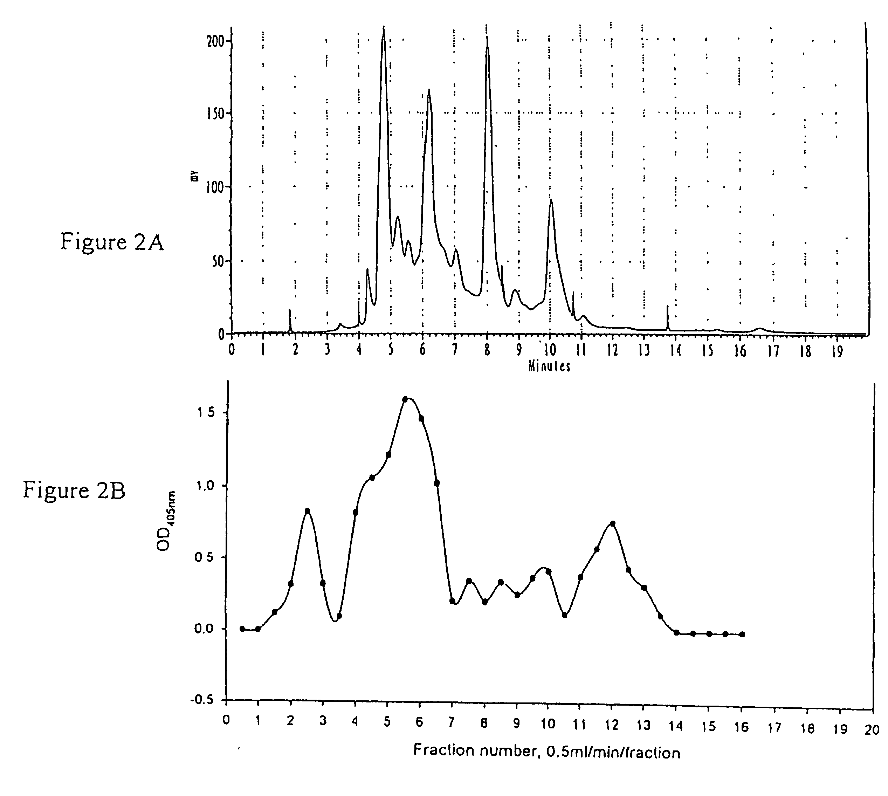

The fractions corresponding to Nos. 25 to 40 in Figure 1A were pooled together, and were injected into the column and analyzed by HPLC in a manner similar to that described in Example 1. The ethanol-water elution was changed to a 20-minute program with an eluant of a linear increase of ethanol concentration from 20 to 30 %. The elution profile was shown in Figure 2A.

The target protein gpIIb/IIIa-binding assay was performed in the same way as described in Example 1. The result indicating the capacity of gpIIb/IIIa binding for each fraction was shown in Figure 2B.

### Example 3

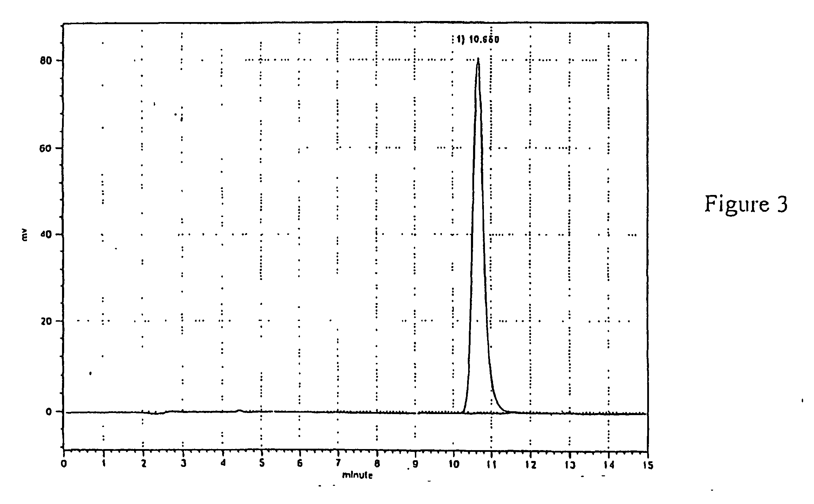

The fractions 5 and 6 shown in Figure 2A were pooled together, for which a third round HPLC analysis was performed with an isocratic eluant containing 9% ethanol and 91% water for 20 minutes. A single peak with a retention time of 10.7 minute showing the strongest binding activity was obtained. The refining profile was shown in Figure 3.

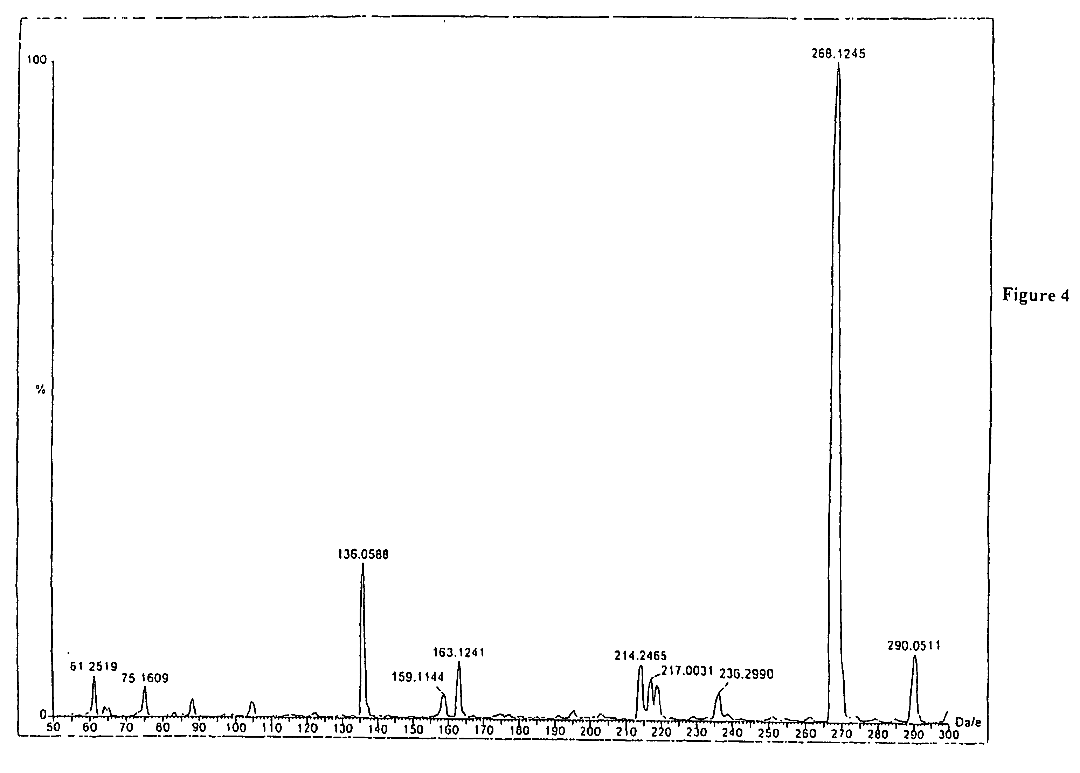

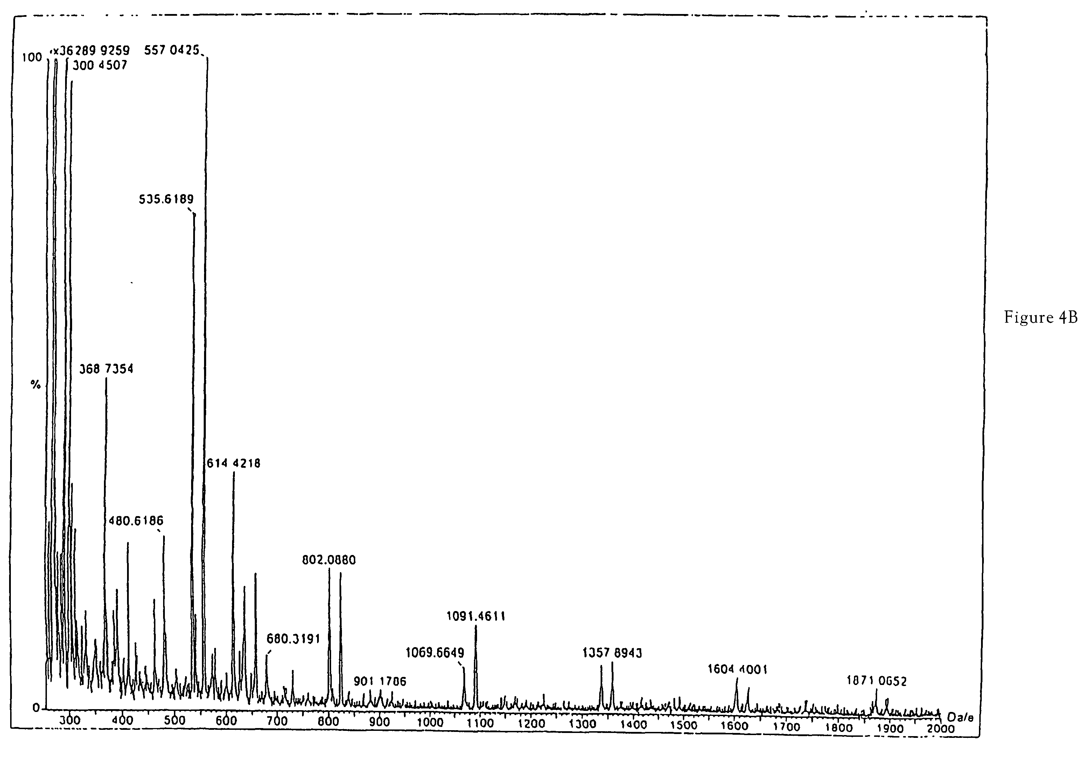

The compound collected from the profile of Figure 3 was dried, and then its molecular weight (MW) was determined by the electrospray ionization mass spectrometry. As shown in Figure 4A, the major peak was determined to be an m/z of 268.04 with a standard deviation of 0.11. Another 289.9 m/z paired peak was also determined by approximately 21.9 m/z apart. It was proposed to be a sodium salt of the major peak. Furthermore, a number of related, multiply-charged and paired peaks (535.6; 802.1; 1069.7; 1357.9; 1604.4; and 1871.1 m/z) were also determined (Figure 4B). Multiply-charged electrospray was often determined for biopolymers, and those peaks shown in Figure 4B were exactly correspondent to dimmer to 7-mer, respectively, indicating that the isolated compound exhibits a polymer-forming capacity. A paired signal by approximately 22 m/z apart associated with each multiplied peak was proposed to be its conjugated sodium salt.

### Example 4

The compound collected from the profile of Figure 3 that was dried in Example 3 was characterized. Its melting point was determined to be 230-232 °C. The elemental analysis yielded the following result: 44.94% C, 4.90% H, 26.21% N and 23.95% O. Its molecular weight was determined by mass spectrometry to be 267.24 g/mole. Form the preliminary data, its molecular formula was calculated to be C₁₀H₁₃N₅O₄.

The techniques of 1-D ¹H and ¹³C NMR, 2-D ¹H-¹H correlation, ¹H-detected ¹³C-¹H, and ¹⁵N-¹H correlation NMR were used to analyze the structure of the compound. The data were collected by using a Varian 600 MHz NMR spectrum. The 1-D ¹H NMR and 2-D ¹H-¹H COSY and TOCSY spectra established the proton connectivity pattern, which revealed that there was a β-ribofuranosyl residue, three exchangeable protons (from OH groups), and a purine ring system. The integration of the fragmentation patterns was consistent with the number of protons from the elemental analysis. The ¹³C NMR analysis data confirmed the number of carbons in the compound. The ¹H-detected ¹³C-¹H NMR was used in the determination of the connection of the carbon atoms with hydrogen atoms with respect to their location. The result was consistent for purine and ribose ring systems. The ¹⁵N-¹H correlation NMR analysis data showed that there were four nitrogen atoms with the pattern consistent with that of a purine ring system. The result obtained was also consistent with that of the elemental analysis. The final experiment that was performed was 1-D ¹H NMR on adenosine, which showed that the spectrum between adenosine and the compound was virtually indistinguishable. The NMR analysis data was shown in the following table:

**Table -**

| ¹H, ¹³C and ¹⁵N chemical shifts (¹H relative to TMS = 0.000 ppm; ¹³C relative to DMSO = 39.80 ppm; and ¹⁵N approximate), and ¹H-¹H coupling constants (> 1.0 Hz; parenthesis) for putative adenosine isomer, the compound collected from the profile of Figure 3, in DMSO-*d*₆ at 308 °K. | | | | |
|---|---|---|---|---|
| | ¹H | (*J*_{i,j}) | ¹³C | ¹⁵N |
| N-1 | - | | | ∼184 |
| H/C-2 | 8.164 | | 152.56 | |
| N-3 | - | | | ∼173 |
| C-4 | - | | 149.39 | |
| C-5 | - | | 119.57 | |
| C-6 | - | | 156.30 | |
| N-7 | - | | | ∼190 |
| H/C-8 | 8.354 | | 140.18 | |
| N-9 | - | | 203.58 | ∼118 |
| H₂N-6 | 7.301 | | | ND |
| H/C-1' | 5.910 | (³*J*_{1,2}=6.3) | 88.18 | |
| H/C-2' | 4.631 | (³*J*_{2,3}=4.9) | 73.64 | |
| H/C-3' | 4.174 | (³*J*_{3,4}=3.4) | 70.81 | |
| H/C-4' | 3.994 | (³*J*_{4,5a}=3.6) | 86.08 | |
| H/C-5a' | 3.574 | (³*J*_{4,5b}=3.4) | 61.83 | |
| H/C-5b' | 3.690 | (³*J*_{5a,5b}=-12.2) | | |
| HO-2' | 5.418 | | | |
| HO-3' | 5.153 | | | |
| HO-5' | 5.381 | | | |

The UV maximum absorbance of the compound was the same as that of adenosine. The IR spectrum of the compound was the same as that of adenosine.

HPLC analysis with two different columns (Synergi Polar-RP and Synergi Max-RP), two different flow rates and several mobile phases and with adenosine as a primary standard showed that the purity of the compound was 95.05% in view of the presence of water in the sample. The retention time for HPLC analysis of the compound and adenosine were identical.

Based on the above analysis, the compound is authentic adenosine.

### Example 5

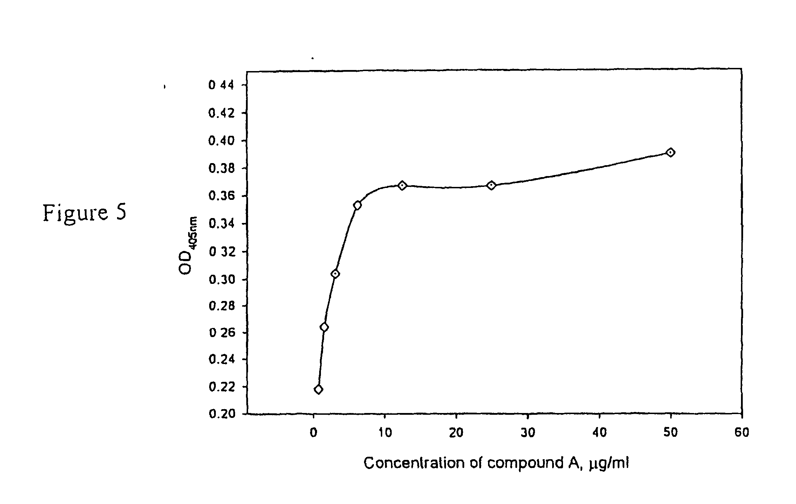

The compound "adenosine" obtained from Example 4 was used as a standard for the characterization of each batch preparation of the compound. The compound, when purified, was collected, dried and used to verify its gpIIb/IIIa binding capacity. For conducting the binding assay, the compound was formulated into an aqueous solution, and a serial dilution for forming a final concentration of 0 to 50 µg/ml was made. Each tested solution contained a different amount of the compound, and was spotted on a flat well of a plastic 96-well plate. Labeled protein gpIIb/IIIa was then added into each tested well, and the coloring process was performed in a way as described in Example 1. The binding curve was shown in Figure 5. It was observed that the binding dramatically increased to as high as 80 % of the maximal binding with the concentration of the compound below 10 µg/ml.

### Example 6

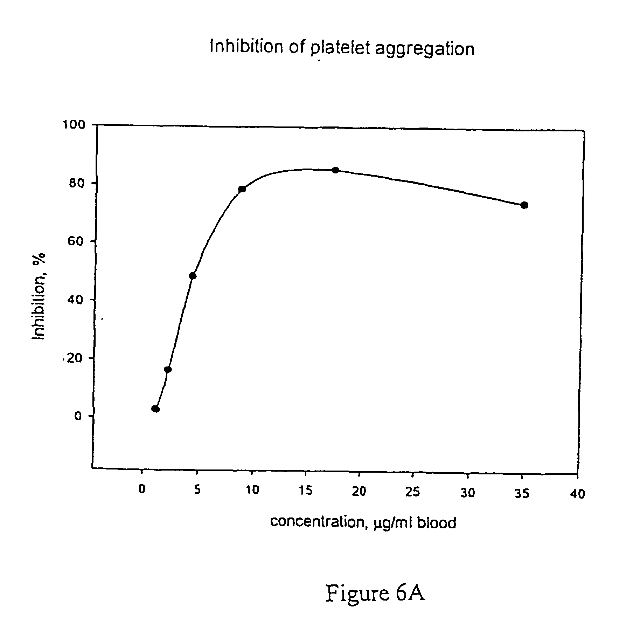

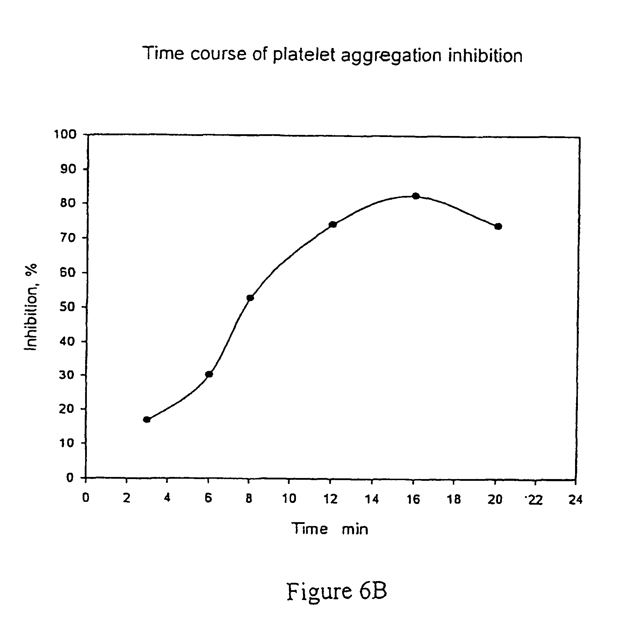

To assay the platelet aggregation-inhibiting activity of the compound, a regular ADP-activated platelet aggregation assay was performed. The reagents used for conducting the assay were purchased from Sigma. Figure 6A showed a dose response of the compound in the inhibition of platelet aggregation. The maximal inhibition activity was about 85 %, which occurs at the concentration of the compound from 10 to 15 µg/ml blood. By using the compound with the concentration of 17.4 µg/ml for determining the inhibition activity on platelet aggregation in a time course manner, Figure 6B revealed that the maximal inhibition activity was about 85 % occurring at the time from the 14^{th} to 16^{th} minutes.

### Example 7

The thrombus formation *in vivo* in mesenteric vein of rats was used for evaluating an effect of the compound on the inhibition of venous thrombosis. In brief, groups of 3 male Wistar-derived rats, each weighing 60±10 gm, were used for each dosage test. The rats were anesthetized with phenobarbital sodium (50 mg/kg, i.p.), and paralyzed with succinylcholine chloride (2 mg/rat, i.p.). A mesenteric loop and vein was then exposed and mounted on a constructed platform. The exposed area was superfused with a normal saline at 37 °C except during electrode placement and stimulation. With the aid of a dissecting microscope and micromanipulator, a monopolar platinum electrode was brought into and contact with the vein. Thrombus formation was initiated by the application of a single square wave electrical pulse (1000 PPS, 100 V, 300 ms) supplied from a grass S-44 stimulator. The formation of thrombus was then observed through the microscope-calibrated ocular lens. The relative venous occlusion (a measured degree of thrombus formation) was determined as a percentage of venous diameter (0.36 to 0.38 mm) and was recorded at 10 seconds (baseline control score) and at 1 minute interval for 20 minutes. The compound or vehicle was administered i.v. for 5 minutes before conducting the electrical stimulation of mesenteric vein. In the vehicle-treated animals, the 20-minute interval recorded values were averaged, which attains a value ranging from 45 to 55% of the interval vein diameter. Consequently, the antithrombic activity of the compound was calculated as % of inhibition relative to the vehicle-treated control animals. If a significant inhibition (>30%) was observed in 3 animals, an ED₃₀±SEM was determined by linear regression using 3 animals per dose level. At each time point, a paired Student's *t* test was applied for the statistical analysis for comparison of the vehicle treatment group with the compound group with a significance referred to as *P<0.05 and **P<0.01.

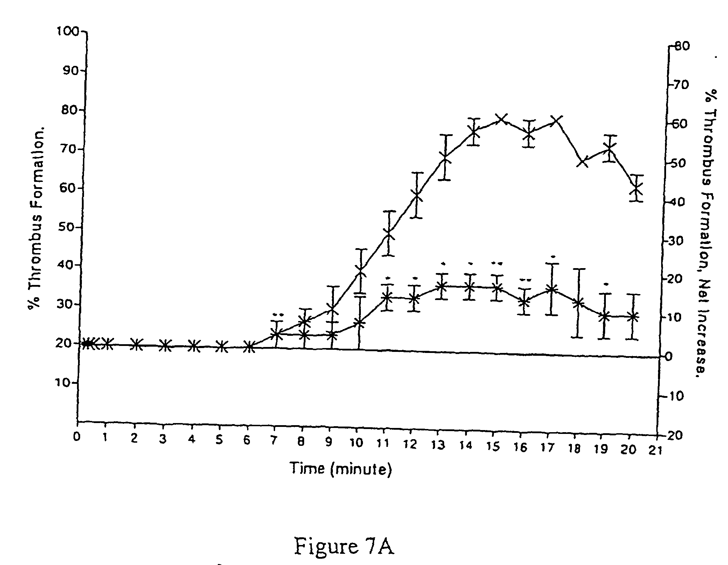

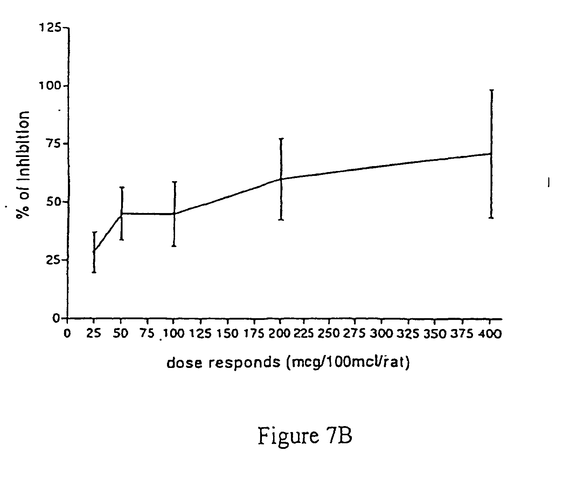

In Figure 7A, the compound exhibiting an antithrombic activity with a concentration of 400 µg/rat revealed significance relative to the vehicle treated group at 7, 11,12, 13, 14, 15, 16, 17, and 19 minutes after inducing an electric stimulation. The antithrombic activity of the compound in a dose response manner in a range of 25, 50, 100, 200 and 400 µg/rat was shown in Figure 7B.

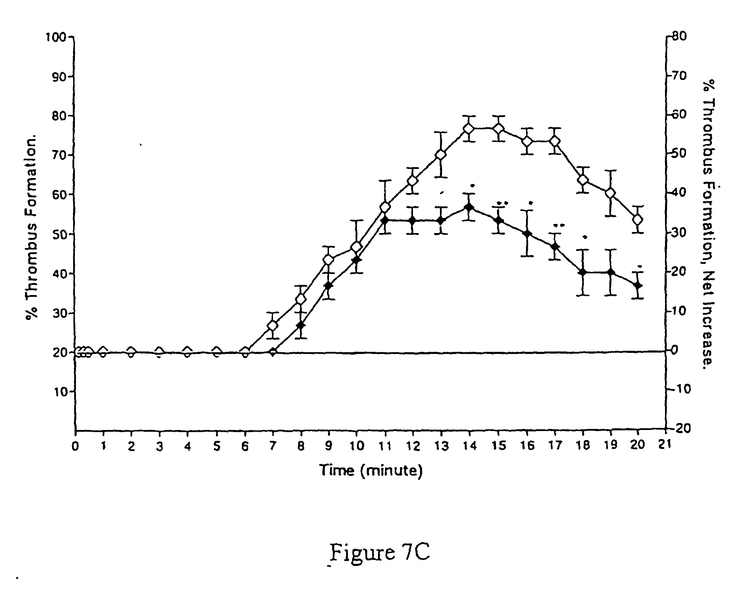

For evaluating the antithrombic activity of the compound by oral administration, an animal model experiment was similarly performed, except that the intravenous administration is replaced with oral feeding at 1 hour prior to the electrical stimulation. A dosage amount of 4 mg/rat was administered. The result was shown in Figure 7C, which revealed that the significant antithrombic activity of the compound group relative to that of the vehicle treated group was observed at 14, 15, 16, 17, 18, and 20 minutes.

### Example 8

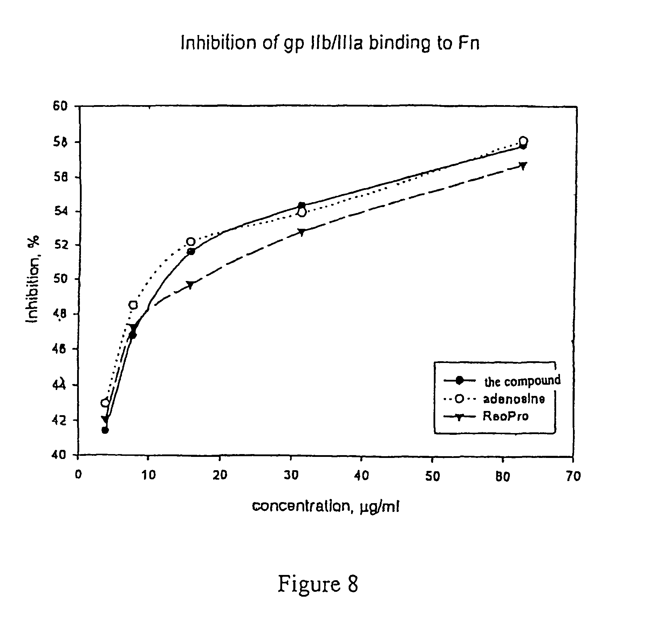

A competitive ELISA assay for analyzing that the binding of fibrinogen to gpIIb/IIIa is inhibited was performed for comparative analysis, where the compound, commercial adenosine (a product from Sigma, Cat. A-9251, Lot. 18H0295) and ReoPro® (abciximab) (10 mg/ml; produced by Eli Lilly for laboratory use only, Lot. 97F03, which is a monoclonal antibody and an antagonist against gpIIb/IIIa) were tested. The gpIIb/IIIa purified from platelet was coated on flat wells of a plastic 96-well plate. Samples of each of the test compounds were prepared in a concentration in a range of 5-60 µg/ml. A solution of biotin-labeled fibrinogen was prepared in a concentration of 1.5 µg/ml. Prior to addition to the wells, each of the samples were individually mixed with an equal volume of the biotinylated fibrinogen. The competitive binding reaction in the plate was performed at 37 °C for 2 hours. After another 2 hours for incubation, strepavidin-conjugated alkaline phosphatase was added to each well of the plate. A substrate solution containing p-nitrophenyl phosphate was then added to each well for coloring by a conventional way. The capability of each tested compound in the inhibition of the binding of fibrinogen to gpIIb/IIIa was measured and recorded as OD value. The result was shown in Figure 8, indicating that the compound and commercial adenosine exhibited a quite similar function in the inhibition of the binding of fibrinogen to gpIIb/IIIa, while the compound exhibited a superior effect in the inhibition of the binding of fibrinogen to gpIIb/IIIa to the ReoPro® (abciximab).

### Example 9

The potential acute toxicity of the compound in adult rats when administered intravenously as a single dose was examined. Twenty animals were divided into five treatment groups of two males and two females each as follows: Group 1: vehicle control; Group 2: 1 mg of the compound/kg body weight of animal; Group 3: 10 mg of the compound/kg body weight of animal; Group 4: 50 mg of the compound/kg body weight of animal; and Group 5: 100 mg of the compound/kg body weight of animal. The tested article was reconstituted with 0.9% sodium chloride injection to provide 0.64±0.05 mg/kg (Group 2), 6.89± 1.13 mg/kg (Group 3), 35.02±5.02 mg/kg (Group 4) and 69.55±5.65 mg/kg (Group 5). Animals were dosed on Day 1, and scheduled necropsies conducted on Day 8 included gross examination of organs and tissues, organ weight measurements, and tissue collection for potential histopathological examination. It was found in the compound treatment groups that there was no change in clinical observations, serum chemistry, hematology, gross examination, body weight and organ weight (absolute and relative) that could be attributed to the compound. No signs of overt toxicity were observed in the compound treatment groups.

## Claims

1. Use of adenosine for the preparation of a medicament for inhibiting thrombosis in mammals.

2. Use of adenosine for the preparation of a medicament for treating mammals for thromboembolic disorders.

3. Use according to claim 2 wherein said thromboembolic disorders are selected from the group consisting of atherosclerosis and arteriosclerosis, acute myocardial infarction, angina, transient ischemic attacks and strokes, peripheral vascular diseases, arterial thrombosis, preeclampsia, embolism and carotid endarterectomy.

## Patentansprüche

1. Verwendung von Adenosin zur Herstellung eines Medikaments zur Inhibierung der Thrombose bei Säugetieren.

2. Verwendung von Adenosin zur Herstellung eines Medikaments zur Behandlung von Säugetieren bei thromboembolischen Erkrankungen.

3. Verwendung nach Anspruch 2, wobei die thromboembolischen Erkrankungen ausgewählt sind aus der Gruppe bestehend aus Atherosklerose und Arteriosklerose, akutem myocardialem Infarkt, Angina, vorübergehenden ischämischen Anfällen und Schlaganfällen, peripheren Gefäßerkrankungen, arterieller Thrombose, Präeklampsie, Embolie und Endarteriektomie der Halsschlagader.

## Revendications

1. Utilisation d'adénosine pour la préparation d'un médicament pour inhiber une thrombose chez les mammifères.

2. Utilisation d'adénosine pour la préparation d'un médicament pour traiter les mammifères souffrant de troubles thromboemboliques.

3. Utilisation selon la revendication 2, dans laquelle lesdits troubles thromboemboliques sont choisis dans le groupe constitué de l'athérosclérose et de l'artériosclérose, de l'infarctus du myocarde en phase aiguë, de l'angine, des attaques ou infarctus ischémiques transitoires, des maladies vasculaires périphériques, de la thrombose artérielle, de la prééclampsie, de l'embolisme et de l'endartériectomie carotidienne.
